(19) Europäisches Patentamt
European Patent Office
Office européen des brevets

(11) Numéro de publication: **0 246 984**
**A2**

(12) # DEMANDE DE BREVET EUROPEEN

(21) Numéro de dépôt: **87420117.1**

(22) Date de dépôt: **04.05.87**

(51) Int. Cl.³: **C 07 D 403/12**
A 01 N 47/36
//C07D271/10, (C07D403/12, 271:00, 239:00), (C07D403/12, 271:00, 251:00)

(30) Priorité: **07.05.86 FR 8606794**
**10.10.86 FR 8614279**

(43) Date de publication de la demande:
**25.11.87 Bulletin 87/48**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon(FR)**

(72) Inventeur: **Borrod, Guy**
**38 Bis rue Des Granges**
**F-69005 Lyon(FR)**

(72) Inventeur: **Guigues, François**
**936 Avenue Victor Hugo**
**F-69140 Rilleux(FR)**

(74) Mandataire: **Ranguis, Patrick Frédéric et al,**
**RHONE POULENC AGROCHIMIE Service DPI B.P. 9163**
**F-69263 Lyon Cédex 09(FR)**

(54) **Herbicides de type sulfonylurée, leur préparation, les compositions les contenant et leur utilisation.**

(57) Herbicides de formule (I):

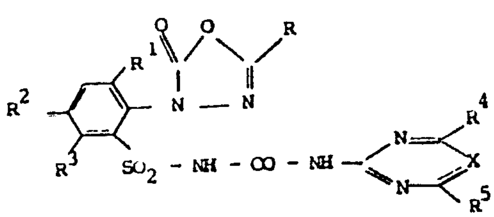

R est l'atome d'hydrogène ou un radical alkyle inférieur, alcényle inférieur, alcynyle inférieur, alcoxy inférieur, alcoxyalkyle inférieur, halogénoalkyle inférieur;

$R_1$, $R_2$, $R_3$ représentent un atome d'hydrogène ou d'halogène ou un radical alkyle inférieur, alcoxy inférieur, halogénoalkyle inférieur, alcoxycarbonyle inférieur;

$R_4$, $R_5$ représentent un radical alkyle inférieur, alcoxy inférieur, halogénoalkyle inférieur (de préférence méthyle ou méthoxy) ou un atome d'halogène;

X est un radical trivalent: –CH= ou –N=.

**EP 0 246 984 A2**

La présente invention concerne de nouvelles sulfonylurées à activité herbicide ainsi que les compositions les contenant, leur procédé de préparation et leur utilisation.

Un but de l'invention est de fournir des composés présentant une activité herbicide.

Un autre but de l'invention est de fournir des composés présentant une activité herbicide sélective vis à vis notamment des céréales, du mais, des betteraves, du tournesol, du riz, du soja, du cotonnier, du colza.

Ces buts sont atteints partiellement ou totalement au moyen des composés de l'invention.

Les nouvelles sulfonylurées selon l'invention ont pour formule la formule (I) donnée ci-après sur page spéciale , dans laquelle :

R est l'atome d'hydrogène ou un radical alkyle inférieur, alcényle inférieur, alcynyle inférieur, alcoxy inférieur, alcoxyalkyle inférieur, halogénoalkyle inférieur ;

$R_1$, $R_2$, $R_3$, identiques ou différents, représentent un atome d'hydrogène ou d'halogène ou un radical alkyle inférieur, alcoxy inférieur, halogénoalkyle inférieur, alcoxycarbonyle inférieur ;

$R_4$, $R_5$, identiques ou différents, représentent un radical alkyle inférieur (de préférence méthyle), alcoxy inférieur (de préférence méthoxy), halogénoalkyle inférieur, un atome d'halogène (de préférence le chlore),

X est un radical trivalent , -CH= ou -N=.

$R_6$ représente l'atome d'hydrogène ou un radical alkyle inférieur (de préférence méthyle), avantageusement $R_6$ est l'atome d'hydrogène.

Selon une première variante avantageuse de l'invention, on choisira pour leurs propriétés herbicides, les composés de formule (I) dans laquelle $R_1$, $R_3$ sont l'atome d'hydrogène.

Selon une seconde variante avantageuse de l'invention prise ou non en combinaison avec la première, on

choisira pour leurs propriétés herbicides les composés de formule (I) dans laquelle R est l'atome d'hydrogène ou un radical alcoxy alkyle inférieur (de préférence méthoxyméthyle) ou alcoxy inférieur (de préférence méthoxy) et X est -CH =.

Selon une troisième variante avantageuse prise ou non en combinaison avec l'une ou deux des précédentes $R_2$ est un atome d'hydrogène, un atome d'halogène (de préférence le chlore), un radical alcoxy inférieur et X est -CH =.

Selon une quatrième variante avantageuse prise ou non en combinaison avec l'une des précédentes $R_4$, $R_5$ identiques ou différents sont un atome d'halogène, ou un radical alkyle inférieur (de préférence méthyle) ou un radical alcoxy inférieur (de préférence méthoxy) et de préférence encore $R_5$ est un radical alkyle inférieur (de préférence méthyle) ou alcoxy inférieur (de préférence méthoxy).

Une autre variante préférée prise ou non en combinaison avec la première variante consiste en ce que $R_1$, $R_2$ sont l'atome d'hydrogène et X est -N = et de préférence $R_4$ est un radical alkyle inférieur (de préférence méthyl) et $R_5$ un radical alcoxy inférieur (de préférence méthoxy).

Dans le présent exposé, l'adjectif inférieur, appliqué à un radical hydrocarboné, signifie qu'il possède au plus 4 atomes de carbone. Les diverses formules (I) et suivantes sont réunies sur une même page qui doit être considérée comme faisant partie intégrante de la description de l'invention et non pas seulement comme des dessins explicatifs.

Les produits selon l'invention de formule (I) peuvent être préparés par réaction d'une amine de formule (II) avec un isocyanate de formule (III). Cet isocyanate de formule (III) peut être préparé par réaction de phosgène sur un sulfonamide de formule (IV), éventuellement en présence d'un initiateur de type alkylisocyanate.

Les produits selon l'invention de formule (I) peuvent également être préparés par réaction d'un carbamate d'aryle de formule (XI) avec un sulfonamide de formule (IV) en présence d'une base forte.

Ce sulfonamide de formule (IV) peut éventuellement être préparé par action d'ammoniac sur un chlorure de sulfonyle de formule (V). Ce chlorure de sulfonyle de formule (V) peut éventuellement être préparé par action de $SO_2$ en milieu acide sur un sel de diazonium dérivé d'amine de formule (VI) laquelle peut éventuellement être préparée par hydrogénation d'un composé nitré de formule (VII).

Ce composé nitré de formule (VII) peut éventuellement être préparé par phosgénation cyclisante d'un composé de type hydrazide de formule (VIII) ou peut être préparé par réaction d'un composé de formule (IX) avec un composé de formule (X).

Dans les diverses formules (II) à (XI), les divers substituants ont mêmes significations que dans la formule (I) ; le symbole Y représente un atome d'halogène, de préférence F ou Cl ; le symbole M représente un atome de métal alcalin, de préférence le lithium, le sodium ou le potassium.

Les divers composés de formules (III) à (XI), et spécialement (III) à (VII), sont des produits nouveaux qui font également partie de l'invention.

L'invention porte encore sur les procédés de préparation des composés de formules (I) et (III) à (VIII).

Les (nitro-2 phényl)-4 oxadiazoline-1,3,4 one -5 de formule (VII) peuvent être obtenues par action du phosgène sur les acyl-1 (nitro -2 phényl)-2 hydrazines de formule (VIII) de préférence en présence d'amine tertiaire, telle que la triéthylamine ou la pyridine, dans un solvant inerte qui peut être par exemple le toluène, le xylène, le chloroforme, le chlorure de méthylène, le dioxanne, le tétrahydrofuranne ; la température de réaction est

généralement comprise entre -78° et 0°C et le rapport molaire ; phosgène : (VIII) entre 1.05 et 5.

Les (nitro-2 phényl)-4 oxadiazoline-1,3,4 ones -5 de formule (VII) peuvent également être obtenues par action des halogéno-1 nitro-2 benzènes de formule (IX), Y correspond à un atome d'halogène, sur les dérivés métalliques des oxadiazoline-1,3-4 ones-5 de formule (X). On opère avantageusement dans un solvant polaire tel que le diméthylformamide, la N-méthyl pyrrolidone-2, le diméthylsulfoxyde ou l'hexaméthylphosphotriamide à une température comprise entre 100 et 200°C et en quantité sensiblement stoechiométrique.

Les oxadiazoline-1,3,4 ones-5 de formule (X) peuvent être préparées par le procédé décrit dans la demande de brevet néerlandais 6500374.

La réduction des dérivés nitrés de formule (VII) en anilines de formule (VI) peut être effectuée :

- soit par des méthodes chimiques bien connues telles que l'action du fer en milieu hydroalcoolique ou hydroacétique, acide ou neutre,
- soit par l'hydrogène en présence d'un catalyseur tel que l'oxyde ou le noir de platine, le palladium sur charbon ou le nickel Raney dans un solvant inerte tel que l'acétate d'éthyle, l'éthanol, l'acide acétique, le tétrahydrofuranne... La pression d'hydrogène peut aller de la pression atmosphérique jusqu'à 100 bars, et la température peut aller de 20 à 50°.

Les anilines de formule (VI) peuvent être transformées en chlorures de sulfonyle (V) par réaction d'un sel (par exemple un chlorure) de diazonium (dérivé d'amine de formule (VII)) sur $SO_2$ de préférence en excés ( 2 à 5 moles pour 1 mole ) en solution dans un solvant tel que les acides alcanoïques inférieurs et les alcanols inférieurs, et en présence d'un sel cuivrique.

Les chlorures de sulfonyle de formule (V) peuvent être transformés en sulfonamides de formule (IV) par action

d'au moins 2 moles d'ammoniac gazeux (anhydre de préférence) dans un solvant inerte tel que le toluène, l'éther éthylique, le dioxanne, le tétrahydrofuranne, le chloroforme, le chlorure de méthylène, à des températures allant de -20° à + 50° et de préférence entre 0 et 10°.

Les sulfonamides de formule (IV) peuvent être transformés en sulfonylisocyanates de formule (III) par action du phosgène de préference en excés (1.5 à 15 moles), en présence d'isocyanate d'alkyle (de préférence de butyle) dans une proportion allant de 10 à 100 % de la stoéchiométrie et en présence d'une quantité catalytique d'une amine tertiaire (par exemple le diaza-1,4 bicyclo-2,2,2 octane) dans un solvant inerte à point d'ébullition élevé tel que : xylène, chlorobenzène, dichlorobenzène, tétraline ; la réaction s'effectue à des températures généralement comprises entre 120 et 200°C.

Le sulfonylisocyanate ainsi obtenu (de formule (III)) peut être lui même transformé, avec ou sans purification, en sulfonylurée de formule (I) par action d'une aminopyrimidine ou d'une aminotriazine de préférence en quantité stoechiométrique dans un solvant inerte tel que chloroforme, acétonitrile, toluène, xylène, chlorobenzène, à une température allant de 20 à 100°.

Les sulfonamides de formules (IV) peuvent être également transformés directement en sulfonylurée de formule (I) par action d'un pyrimidinyl- ou triazinyl carbamate d'aryle (XI), (de préférence phényle), de préférence en quantité stoechiométrique en présence d'une amine tertiaire (par exemple le diaza-1,8 bicyclo [5,4,0] undecène -7) de préférence également en quantité stoechiométrique dans un solvant inerte tel que chloroforme, acétonitrile, acétone, tétrahydrofuranne ou dioxanne à une température allant de 20 à 100°.

Le carbamate d'aryle (XI) peut être obtenu par action d'un chlorofomiate d'aryle sur une amine de formule (II) en présence d'une amine tertiaire telle que la

pyridine en solvant polaire aprotique tel que le tétrahydrofuranne à température ambiante de préférence.

Les exemples suivants donnés à titre non limitatif illustrent l'invention et montrent comment elle peut être mise en pratique. Les produits préparés et utilisés dans ces exemples ont l'une ou l'autre des formules (I) à (X).Les exemples 1 à 5 illustrent la préparation des produits ; les exemples 6 et 7 illustrent son utilisation ; les termes de prélevée et de préémergence sont synonymes ; les termes de postlevée et de postémergence sont synonymes ; les résultats obtenus au cours des exemples 6 et 7 sont réunis sous forme de tableau situé après l'exemple 5.

Exemple 1 :

Préparation du N-(diméthyl-4,6 pyrimidinyl-2) amino carbonyl (oxadiazoline-1,3,4 one -5 yl -4)-2 benzène sulfonamide (produit inclus dans la formule (I).

Un courant de phosgène (7 g/heure) est envoyé dans une solution de 27,3 g (0,1132 mole) d'oxadiazoline-1,3,4 one -5 yl -4)-2 benzène sulfonamide (produit inclus dans la formule (IV)), 11,2 g (0,1132 mole) d'isocyanate de butyle et 0,6 g de diazabicyclo-2,2,2 octane dans 340 ml de xylène portée à 130-135° pendant 12 heures. On refroidit, filtre et le produit filtré est lavé avec 150 ml de chloroforme. Le filtrat et le chloroforme de lavage sont évaporés à sec et le résidu repris par 150 ml de chloroforme et ajouté à une solution de 14,0 g (0,1132 mole) d'amino-2 diméthyl-4,6 pyrimidine dans 50 ml de chloroforme. La température s'élève à 28°. On agite pendant 4 heures à température ambiante, évapore le solvant et reprend par 80 ml d'acétonitrile le résidu qui est filtré et lavé avec de l'acétonitrile. Après séchage, on obtient 9,1 g de cristaux de couleur blanche fondant à 225°C avec décomposition (Rendement = 20,6 % ; Produit inclus dans la formule (I)).

<u>Préparation de l'(oxadiazoline-1,3,4 one -5 yl -4)-2 benzène sulfonamide</u> (produit inclus dans la formule (IV)).

5 g (0,292 mole) d'ammoniac gazeux sont dissous dans une solution de 38,1 g (0,146 mole) d'(oxadiazoline-1,3,4 one -5 yl -4)-2 benzène sulfochlorure (ou chlorure de sulfonyle ; produit inclus dans la formule (V)) dans 438 ml de chloroforme à 5°. Le milieu est agitépendant 1 heure à 5° puis évaporé à sec. Le résidu est repris par 500 ml d'eau. Les cristaux sont filtrés et lavés à l'eau puis séchés, donnant 27,3 g de cristaux blancs fondant à 142° (Rendement = 77,6 %).

<u>Préparation de l'(oxadiazoline-1,3,4 one -5 yl -4)-2 benzène sulfochlorure</u> (produit inclus dans la formule (V).

Une solution de 31,8 g (0,179 mole) d'(amino-2 phényl)-4 oxadiazoline-1,3,4 one -5 (produit inclus dans la formule (VI)) dans 63,6 ml d'acide acétique est coulée goutte à goutte dans 318 ml d'acide chlorhydrique concentré à -5°. Puis une solution de 12,8 g (0,185 mole) de nitrite de sodium dans 38 ml d'eau est ajoutée goutte à goutte dans le mélange, en 20 minutes, en maintenant la température à -5°. L'agitation est ensuite poursuivie pendant une heure à la même température puis 2,7 g d'urée sont ajoutés en une fois. Dix minutes après, le milieu est filtré puis coulé rapidement dans une solution saturée d'anhydride sulfureux dans 360 ml d'acide acétique à laquelle on a ajouté une solution de 4,9 g de chlorure cuivrique bihydraté dans 41,6 ml d'eau. Le mélange est porté à 40°. Lorsque le dégagement gazeux cesse, le milieu est versé dans 1 200 ml d'eau. Le précipité blanc formé est filtré, lavé à l'eau et séché. On obtient 38,1 g de cristaux blancs fondant à 144° (Rendement = 81,5 %).

<u>Préparation d'(amino-2 phényl)-4 oxadiazoline-1,3,4 one -5</u> (produit inclus dans la formule (VI)).

19,7 g (0,095 mole) de (nitro-2 phényl)-4 oxadiazoline -1,3,4 one -5 (produit inclus dans la formule

(VII)) sont placés dans un autoclave de 1 l avec 394 ml d'acétate d'éthyle et 3,8 g de charbon palladié ( à 5 % de Palladium et 50 % d'eau) sous une pression de 40 bars d'hydrogène et sous agitation. La température s'élève de 18 à 42°. Au bout de 3 heures la pression d'hydrogène est stabilisée. Au total 15 bars d'hydrogène ont été absorbés. Après retour à la pression atmosphérique, le mélange est filtré et le filtrat évaporé sous vide à 50°. Le résidu cristallisé est malaxé avec 150 ml d'oxyde d'isopropyle puis filtré et séché. On obtient 16,5 g de cristaux de couleur blanc crème (Rendement = 98,0 %) fondant à 78,3°C.

Préparation de la (nitro-2 phényl)-4 oxadiazoline-1,3,4 one -5 (produit inclus dans la formule (VII)).

61,6 g (0,623 mole) de phosgène sont dissous dans 1167 ml de toluène à 0°. Le mélange est refroidi à -20° et 174,3 ml (126 g , 1,245 mole) de triéthylamine sont coulés en 20 minutes. 12,45 g (0,05 mole) de formyl-1 (nitro-2 phényl)-2 hydrazine sont additionnés rapidement. La suspension brune obtenue est agitée pendant 4 heures à -20°. On coule alors 390 ml d'eau dans le milieu réactionnel à la température de 0°. Après 15 minutes d'agitation suivie d'une décantation, la phase organique est lavée à l'eau puis séchée. Après évaporation du toluène, on obtient 20,4 g. d'un solide qui est purifié par passage sur gel de silice : 13,7 g (Rendement : 42,5 %) de cristaux de couleur rouge orangé fondant à 70,7°C.

Exemple 2 : Préparation du N-(méthyl-4 méthoxy-6 triazine-1,3,5 yl-2) amino carbonyl (oxadiazoline-1,3,4 one-5 yl-4)-2 benzène sulfonamide (produit inclus dans la formule (I))

On opère comme à l'exemple 1 avec les variantes suivantes :

On remplace les 14,0 g d'amino-2-diméthyl-4,6 pyrimidine par 15,9 g d'amino-2-méthyl-4-méthoxy-6 triazine

et on obtient 12,1 g de produit inclus dans la formule (I) (Rendement = 26,2 %) fondant à 201°C.

Exemple 3 :

Préparation du N-(chloro-4 méthoxy-6 pyrimidinyl-2) amino carbonyl (oxadiazoline-1,3,4 one -5 yl -4)-2 benzène sulfonamide (produit inclus dans la formule (I).

Un courant de phosgène (7 g/heure) est envoyé dans une solution de 6,5 g (0,027 mole) d'oxadiazoline-1,3,4 one -5 yl -4)-2 benzène sulfonamide (produit inclus dans la formule (IV)), 3,0 g (0,027 mole) d'isocyanate de butyle et 0,15 g de diazabicyclo-2,2,2 octane dans 80 ml de xylène portée à 130-135°C pendant 15 heures. On filtre le milieu à chaud (80 à 100°C). Le filtrat est évaporé à sec et le résidu lavé à l'heptane, puis repris par 100 ml de dichlorométhane et ajouté à une solution de 4,3 g (0,027 mole) d'amino-2 chloro-4 méthoxy-6 pyrimidine dans 50 ml de dichlorométhane. On agite pendant 48 heures à température ambiante, filtre le produit cristallisé formé et le lave avec du dichlorométhane. Après séchage, on obtient 1,1 g de cristaux de couleur blanche fondant à 188°C avec décomposition (Rendement = 9,6 % ; Produit inclus dans la formule (I)).

Préparation de l'(oxadiazoline-1,3,4 one -5 yl -4)-2 benzène sulfonamide (produit inclus dans la formule (IV)).

5 g (0,292 mole) d'ammoniac gazeux sont dissous dans une solution de 38,1 g (0,146 mole) d'(oxadiazoline-1,3,4 one -5 yl -4)-2 benzène sulfochlorure (ou chlorure de sulfonyle ; produit inclus dans la formule (V)) dans 438 ml de chloroforme à 5°. Le milieu est agitépendant 1 heure à 5° puis évaporé à sec. Le résidu est repris par 500 ml d'eau. Les cristaux sont filtrés et lavés à l'eau puis séchés, donnant 27,3 g de cristaux blancs fondant à 142° (Rendement = 77,6 %).

<u>Préparation de l'(oxadiazoline-1,3,4 one -5 yl -4)-2 benzène sulfochlorure</u> (produit inclus dans la formule (V).

Une solution de 31,8 g (0,179 mole) d'(amino-2 phényl)-4 oxadiazoline-1,3,4 one -5 (produit inclus dans la formule (VI)) dans 63,6 ml d'acide acétique est coulée goutte à goutte dans 318 ml d'acide chlorhydrique concentré à -5°. Puis une solution de 12,8 g (0,185 mole) de nitrite de sodium dans 38 ml d'eau est ajoutée goutte à goutte dans le mélange, en 20 minutes, en maintenant la température à -5°. L'agitation est ensuite poursuivie pendant une heure à la même température puis 2,7 g d'urée sont ajoutés en une fois. Dix minutes après, le milieu est filtré puis coulé rapidement dans une solution saturée d'anhydride sulfureux dans 360 ml d'acide acétique à laquelle on a ajouté une solution de 4,9 g de chlorure cuivrique bihydraté dans 41,6 ml d'eau. Le mélange est porté à 40°. Lorsque le dégagement gazeux cesse, le milieu est versé dans 1 200 ml d'eau. Le précipité blanc formé est filtré, lavé à l'eau et séché. On obtient 38,1 g de cristaux blancs fondant à 144° (Rendement = 81,5 %).

<u>Préparation d'(amino-2 phényl)-4 oxadiazoline-1,3,4 one -5</u> (produit inclus dans la formule (VI)).

19,7 g (0,095 mole) de (nitro-2 phényl)-4 oxadiazoline -1,3,4 one -5 (produit inclus dans la formule (VII)) sont placés dans un autoclave de 1 l avec 394 ml d'acétate d'éthyle et 3,8 g de charbon palladié ( à 5 % de Palladium et 50 % d'eau) sous une pression de 40 bars d'hydrogène et sous agitation. La température s'élève de 23 à 42°. Au bout de 3 heures la pression d'hydrogène est stabilisée. Au total 15 bars d'hydrogène ont été absorbés. Après retour à la pression atmosphérique, le mélange est filtré et le filtrat évaporé sous vide à 50°. Le résidu cristallisé est malaxé avec 150 ml d'oxyde d'isopropyle puis filtré et séché. On obtient 16,5 g de cristaux de couleur blanc crème (Rendement = 98,0 %) fondant à 78,3°C.

Préparation de la (nitro-2 phényl)-4 oxadiazoline-1,3,4 one -5 (produit inclus dans la formule (VII)).

61,6 g (0,623 mole) de phosgène sont dissous dans 1167 ml de toluène à 0°. Le mélange est refroidi à -20° et 174,3 ml (126 g , 1,245 mole) de triéthylamine sont coulés en 20 minutes. 12,45 g (0,05 mole) de formyl-1 (nitro-2 phényl)-2 hydrazine sont additionnés rapidement. La suspension brune obtenue est agitée pendant 4 heures à -20°. On coule alors 390 ml d'eau dans le milieu réactionnel à la température de 0°. Après 15 minutes d'agitation suivie d'une décantation, la phase organique est lavée à l'eau puis séchée. Après évaporation du toluène, on obtient 20,4 g. d'un solide qui est purifié par passage sur gel de silice : 13,7 g (Rendement : 42,5 %) de cristaux de couleur rouge orangé fondant à 70,7°C.

Exemple 4 :
N-(chloro-4 méthyl-6 pyrimidinyl-2) amino carbonyl (oxadiazoline-1,3,4 one-5 yl-4)-2 benzène sulfonamide.

On opère comme à l'exemple 1 avec les variantes suivantes :

On remplace les 4,3 g (0,027 mole) d'amino-2 chloro-4- méthoxy-6 pyrimidine par 3,86 g (0,027 mole) d'amino-2 chloro-4 méthyl-6 pyrimidine et on obtient 2,5 g de cristaux de couleur blanche (Rendement = 22,7 %) fondant à 209°C avec décomposition.Produit inclus dans la revendication 1.

Exemple 5 : N-(diméthoxy-4,6 pyrimidinyl-2) amino carbonyl (oxadiazoline-1,3,4 one-5 yl-4)-2 benzène sulfonamide (produit inclus dans la formule (I)).

On opère comme à l'exemple 1 avec les variantes suivantes : on remplace les 4,3 g (0,027 mole) d'amino-2 chloro-4 méthoxy-6 pyrimidine par 4,17 g (0,027 mole) d'amino-2 diméthoxy 4,6 pyrimidine et on obtient 2,4 g de

cristaux de couleur blanche fondant à 208°C avec décomposition (Rendement = 21,0 % ; produit inclus dans la formule (I')).

Exemple 6 : N-(méthyl-4 méthoxy-6 pyrimidinyl-2) amino carbonyl (oxadiazoline-1,3,4 one-5 yl-4)-2 benzène sulfonamide (produit inclus dans la formule (I)).

On opère comme à l'exemple 1 avec les variantes suivantes : on remplace les 4,3 g (0,027 mole) d'amino-2 chloro-4 méthoxy-6 pyrimidine par 3,74 g (0,027 mole) d'amino-2 méthyl-4 méthoxy-6 pyrimidine et on obtient 5,6 g de cristaux de couleur jaune pâle fondant à 186°C avec décomposition (Rendement = 51,4 % ; produit inclus dans la formule (I)).

Exemple 7 : N-(diméthoxy-4,6 triazine-1,3,5 yl-2) aminocarbonyl (oxadiazoline-1,3,4 one-5 yl-4)-2 benzène sulfonamide (produit inclus dans la formule (I))

On opère comme à l'exemple 1 avec les variantes suivantes : on remplace les 4,3 g (0,027 mole) d'amino-2 chloro-4 méthoxy-6 pyrimidine par 4,2 g (0,027 mole) d'amino-2 diméthoxy-4,6 triazine-1,3,5 et on obtient 2,4 g de cristaux de couleur blanche fondant à 198°C avec décomposition (Rendement = 21,0 % ; produit inclus dans la formule (I)).

De la même manière on prépare les produits 8 à 17, tous inclus dans la formule (I), les radicaux R, $R_1$, $R_2$, $R_3$, $R_4$, $R_5$ et X étant ceux de la formule (I) avec $R_1$ = $R_3$ = H et X = CH. Les caractéristiques de ces produits sont résumés dans le tableau ci-après qui, de plus, dans un but de clarté inclut les produits 1 à 7 et 18, 19.

Exemple 18 : N-(diméthoxy-4,6 pyrimidinyl-2) amino carbonyl (oxadiazoline -1,3,4 one -5 yl -4)-2 chloro-3 benzène sulfonamide (produit inclus dans la formule I).

3,3 g (0,012 mole) d'(oxadiazoline -1,3,4 one -5 yl-4)-2 chloro-3 benzène sulfonamide, 3,3 g (0,012 mole) de (diméthoxy -4,6 pyrimidinyl-2) carbamate de phényle et 1,8 g (0,012 mole) de diaza-1,8 bicyclo [5,4,0] undécène -7 sont mis en suspension dans 60 ml d'acétonitrile anhydre et agités pendant 3 heures à température ambiante.

Le milieu réactionnel est coulé lentement dans 120 ml de solution normale d'acide chlorhydrique. Les cristaux formés sont filtrés, lavés à l'eau et séchés ; on obtient 1,9 g de cristaux de couleur blanche fondant à 193° (rendement = 34,5 %).

Exemple 19 : N-(diméthyl-4,6 pyrimidinyl-2) amino carbonyl (méthoxy-2 oxadiazoline -1,3,4 one -5 yl -4)-2 benzène sulfonamide (produit inclus dans la formule I).

3,25 g (0,012 mole) de (méthoxy-2 oxadiazoline-1,3,4 one -5 yl-4)-2 benzène sulfonamide, 2,9 g de (diméthyl-4,6 pyrimidinyl-2) carbamate de ohényle et 1,8 g (0,012 mole) de diaza-1,8 bicyclo [5,4,0] undécène-7 sont mis en suspension dans 50 ml d'acétonitrile et traités comme à l'exemple précédent.

On obtient : 0,5 g de cristaux de couleur blanche fondant avec décomposition à 208°C (rendement = 10 %).

| Ex | R | $R_1$ | $R_2$ | $R_3$ | $R_4$ | $R_5$ | F°C | Rdt % | X |
|---|---|---|---|---|---|---|---|---|---|
| 1 | H | H | H | H | Me | Me | 225 | 20,6 | -CH = |
| 2 | H | H | H | H | OMe | Me | 201 | 26,2 | -N = |
| 3 | H | H | H | H | Cl | OMe | 188 | 9,6 | -CH = |
| 4 | H | H | H | H | Cl | Me | 209 | 22,7 | -CH = |
| 5 | H | H | H | H | OMe | OMe | 208 | 21 | -CH = |
| 6 | H | H | H | H | Me | OMe | 186 | 51,4 | -CH = |
| 7 | H | H | H | H | OMe | OMe | 198 | 21 | -N = |
| 8 | H | H | Cl | H | Me | Me | dec. 190-5° | 18,2 | -CH = |
| 9 | H | H | Cl | H | OMe | OMe | 181-2 | 33,1 | -CH = |
| 10 | H | H | Cl | H | Cl | OMe | 183 | 6,8 | -CH = |
| 11 | H | H | Cl | H | Me | OMe | 189 | 15,9 | -CH = |
| 12 | MeOMe | H | H | H | OMe | OMe | 206-7 | 55,6 | -CH = |
| 13 | H | H | OMe | H | Me | Me | 206-7 | 25,6 | -CH = |
| 14 | H | H | OMe | H | Me | OEt | 187-8 | 15,4 | -CH = |
| 15 | H | H | OMe | H | OMe | OMe | 213 | 34,9 | -CH = |
| 16 | H | H | OMe | H | Cl | OMe | 203,5 | 27 | -CH = |
| 17 | H | H | OMe | H | Me | OMe | 201,7 | 16,8 | -CH = |
| 18 | H | Cl | H | H | OMe | OMe | 193 | 34,5 | -CH = |
| 19 | OMe | H | H | H | Me | Me | 208 | 10 | -CH = |

Exemple 20 : Application herbicide, en prélevée des espèces végétales

Dans des pots de 7 x 7 x 8 cm remplis de terre agricole légère, on sème un nombre de graines déterminé en fonction de l'espèce végétale et de la grosseur de la graine.

Les pots sont traités par pulvérisation de bouillie en quantité correspondant à une dose volumique d'application de 500 l/ha et contenant la matière active à la concentration désirée.

Le traitement avec la bouillie est donc effectué sur des graines non recouvertes de terre (on utilise le terme de bouillie pour désigner, en général, les compositions diluées à l'eau, telles qu'on les applique sur les végétaux).

La bouillie utilisée pour le traitement est une suspension aqueuse de la matière active contenant 0,1 % en poids de Cémulsol NP 10 (agent tensio-actif constitué d'alkylphénol polyéthoxylé, notamment de nonylphénolpolyéthoxylé) et 0,04 % en poids de Tween 20 (agent tensio-actif constitué d'un oléate de dérivé polyoxyéthylèné du sorbitol).

Cette suspension a été obtenue en mélangeant et broyant les ingrédients dans un microniseur, de façon à obtenir une grosseur moyenne de particules inférieure à 40 microns.

Différentes concentrations en matière active de la bouillie ont été utilisées qui correspondaient à différentes doses de matière active appliquée. Les résultats sont présentés pour une dose d'application de 125g/ha.

Après traitement, on recouvre les graines d'une couche de terre d'environ 3 mm d'épaisseur.

Les pots sont alors placés dans des bacs destinés à recevoir l'eau d'arrosage, en subirrigation, et maintenus

pendant 24 jours à température ambiante sous 70 % d'humidité relative.

Au bout de 24 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin. Les résultats obtenus sont présentés après l'exemple 21.

<u>Exemple 21</u> : <u>Application herbicide, en postlevée des espèces végétales</u>.

Dans des pots de 7 x 7 x 8 cm remplis de terre agricole légère, on sème un nombre de graines déterminé en fonction de l'espèce végétale et de la grosseur de la graine.

On recouvre ensuite les graines d'une couche de terre d'environ 3 mm d'épaisseur et on laisse germer la graine jusqu'à ce qu'elle donne naissance à une plantule au stade convenable. Le stade de traitement pour les graminées est le stade "deuxième feuille en formation". Le stade de traitement pour les dicotylédones, est le stade "cotylédons étalés, première feuille vraie en développement".

Les pots sont alors traités par pulvérisation de bouillie en quantité correspondant à une dose volumique d'application de 500 l/ha et contenant la matière active à la concentration désirée.

La bouillie a été préparée de la même manière qu'à l'exemple 20.

Différentes concentrations en matière active de la bouillie ont été utilisées qui correspondaient à différentes doses de matière active appliquée. Les résultats sont présentés pour une dose d'application de 125g/ha.

Les pots traités sont ensuite placés dans des bacs destinés à recevoir l'eau d'arrosage, en subirrigation, et maintenus pendant 24 jours à température ambiante sous 70 % d'humidité relative.

Au bout de 24 jours, on compte le nombre de plantes vivantes dans les pots traités par la bouillie contenant la matière active à tester et le nombre de plantes vivantes dans un pot témoin traité selon les mêmes conditions, mais au moyen d'une bouillie ne contenant pas de matière active. On détermine ainsi le pourcentage de destruction des plantes traitées par rapport au témoin non traité. Un pourcentage de destruction égal à 100 % indique qu'il y a eu destruction complète de l'espèce végétale considérée et un pourcentage de 0 % indique que le nombre de plantes vivantes dans le pot traité est identique à celui dans le pot témoin.

Les espèces végétales utilisées dans ces exemples 20 et 21 sont :

| Abreviations | Nom latin | Nom français |
|---|---|---|
| AVE | Avena fatua | Folle avoine |
| ECH | Echinochloa crusgalli | Panisse |
| LOL | Lolium multiflorum | Ray-grass |
| CYP | Cyperus esculentus | Cyperus |
| CEN | Centaurea cyanus | Centaurée |
| IPO | Ipomea purpurea | Liseron pourpre |
| SIN | Sinapis arvensis | Moutarde |
| ABU | Abutilon theophrasti | Abutilon |
| DAU | Daucus carota | Carotte |
| PHS | Phaseolus vulgaris | Haricot |

Les résultats obtenus en prélevée (mode d'application de l'exemple 20) sont les suivants :

| EX | AVE | ECH | LOL | CYP | CEN | IPO | SIN | ABU | DAU | PHS |
|---|---|---|---|---|---|---|---|---|---|---|
| 1 | 0 | 100 | 100 | - | 80 | 20 | 80 | 100 | 90 | 80 |
| 2 | 0 | 100 | 100 | - | 80 | 30 | 30 | - | 0 | 90 |
| 3 | 80 | 100 | 100 | 100 | 90 | 90 | 100 | 95 | 95 | 50 |
| 4 | 0 | 0 | 80 | 100 | 30 | 30 | 30 | 50 | 50 | 0 |
| 5 | 80 | 100 | 100 | 100 | 100 | 50 | 100 | 100 | 100 | 50 |
| 6 | 30 | 100 | 100 | 100 | 95 | 70 | 100 | 100 | 100 | 50 |
| 7 | 20 | 100 | 100 | 90 | 50 | 0 | 90 | 80 | 90 | 100 |
| 8 | 30 | 100 | 98 | 100 | 80 | 60 | 90 | 40 | 30 | 30 |
| 9 | 20 | 100 | 98 | 100 | 70 | 0 | 90 | 80 | 20 | 60 |
| 10 | 20 | 98 | 98 | 100 | 30 | 0 | 80 | 20 | 20 | 30 |
| 11 | 30 | 100 | 98 | 95 | 0 | 0 | 60 | 20 | 30 | 30 |
| 12 | 20 | 100 | 100 | 100 | 30 | 30 | 80 | 98 | 30 | 0 |
| 13 | 0 | 100 | 100 | 100 | 100 | 70 | 98 | 50 | 90 | - |
| 14 | 0 | 100 | 100 | 100 | 0 | 0 | 95 | 0 | 80 | - |
| 15 | 0 | 100 | 100 | 100 | 30 | 90 | 90 | 80 | 80 | - |
| 16 | 0 | 100 | 100 | 100 | 50 | 30 | 50 | 30 | 80 | - |
| 17 | 0 | 100 | 100 | 100 | 50 | 80 | 80 | 80 | 90 | - |
| 18 | 0 | 100 | 100 | 100 | 80 | 100 | 98 | 0 | 100 | - |
| 19 | 0 | 90 | 80 | 100 | 0 | 0 | 90 | 0 | 50 | - |

Les résultats obtenus en postlevée (mode d'application de l'exemple 21) sont les suivants :

| EX | AVE | ECH | LOL | CYP | CEN | IPO | SIN | ABU | DAU | PHS |
|---|---|---|---|---|---|---|---|---|---|---|
| 1 | 100 | 100 | 100 | - | 50 | 100 | 100 | 100 | 100 | 100 |
| 2 | 100 | 100 | 100 | - | 80 | 100 | 100 | 100 | 100 | 100 |
| 3 | 100 | 100 | 100 | 70 | 50 | 100 | 100 | 100 | 100 | 100 |
| 4 | 0 | 0 | 80 | 100 | 30 | 30 | 30 | 50 | 50 | 0 |
| 5 | 100 | 100 | 100 | 30 | 100 | 100 | 100 | 100 | 100 | 100 |
| 6 | 100 | 100 | 100 | 0 | 30 | 100 | 100 | 100 | 100 | 100 |
| 7 | 0 | 100 | 100 | 30 | 0 | 80 | 100 | 20 | 100 | 100 |
| 8 | 0 | 80 | 30 | - | 40 | 60 | 95 | 90 | 95 | 0 |
| 9 | 40 | 98 | 98 | - | 50 | 80 | 100 | 100 | 100 | 95 |
| 10 | 20 | 95 | 40 | - | 50 | 40 | 100 | 95 | 100 | 50 |
| 11 | 60 | 80 | 80 | - | 20 | 50 | 95 | 0 | 95 | 20 |
| 12 | 90 | 100 | 100 | 0 | 30 | 100 | 100 | 100 | 100 | 100 |
| 13 | 90 | 100 | 98 | 0 | 90 | 100 | 100 | 90 | 100 | - |
| 14 | 20 | 95 | 80 | 0 | 0 | 100 | 100 | 100 | 100 | - |
| 15 | 100 | 100 | 100 | 70 | 30 | 100 | 100 | 30 | 100 | - |
| 16 | 100 | 100 | 100 | 0 | 30 | 100 | 100 | 20 | 100 | - |
| 17 | 30 | 100 | 80 | 0 | 0 | 100 | 100 | 30 | 100 | - |
| 18 | 30 | 100 | 80 | 0 | 80 | 80 | 100 | 90 | 100 | - |
| 19 | 0 | 0 | 30 | 0 | 0 | 0 | 100 | 0 | 50 | - |

Les essais réalisés montrent donc les propriétés remarquablement avantageuses des composés selon l'invention, aussi bien pour des traitements en pré-levée des cultures, que pour les traitements en post-levée, et cela aussi bien sur des adventices monocotylédones (graminées) que sur des dicotylédones.

Pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides, fongicides ou herbicides) ou à propriétés régulatrices de la croissance des plantes. Plus généralement les composés utilisés dans l'invention peuvent être associés à tous les additifs solides ou liquides correspondant aux techniques habituelles de la mise en formulation.

Les doses d'emploi des composés utilisés dans l'invention peuvent varier dans de larges limites, notamment selon la nature des mauvaises herbes à éliminer et le degré d'infestation habituel des cultures pour ces mauvaises herbes.

D'une façon générale, les compositions selon l'invention contiennent habituellement de 0,05 à 95 %

environ (en poids) d'une ou plusieurs matières actives selon l'invention, de 1 % à 95 % environ de un ou plusieurs supports solides ou liquides et éventuellement de 0,1 à 50% environ de un ou plusieurs agents tensioactifs.

Selon ce qui a déjà été dit les composés utilisés dans l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol ; esters, notamment l'acétate de méthyl-glycol ; cétones, notamment la cyclohexanone et l'isophorone ; fractions de pétrole ; hydrocarbures aromatiques, notamment les xylènes, ou paraffiniques ; hydrocarbures chlorés aliphatiques, notamment le trichloroéthane, ou aromatiques, notamment les chlorobenzènes ; des solvants hydrosolubles tels que le diméthylformamide, le diméthylsulfoxyde, la N-méthyl-pyrrolidone ; gaz liquéfiés, etc..).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters

phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Comme formes de compositions liquides ou destinées à constituer des compositions liquides lors de l'application, on peut citer les solutions, en particulier les émulsions, les suspensions concentrées, les aérosols, les poudres mouillables (ou poudre à pulvériser), les granulés autodispersibles et les pâtes.

Les suspensions concentrées, qui sont applicables en pulvérisation, sont préparées de manière à obtenir un produit fluide stable ne se déposant pas (broyage fin) et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu soluble ou non soluble : certaines matières solides organiques ou des sels minéraux peuvent être dissous dans le support pour aider à empêcher

la sédimentation ou comme antigels pour l'eau.

A titre d'exemple, voici une composition de suspension concentrée (Exemple 22) :

- matière active 250 g
- phosphate de tristyrylphénol polyéthoxylé 50 g
- alkylphénol polyéthoxylé 50 g
- polycarboxylate de sodium 20 g
- éthylène glycol 50 g
- huile organopolysiloxanique (antimousse) 1 g
- polysaccharide 1,5 g
- eau 566,5 g

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quant c'est nécessaire, de 0 à 10 % d'un ou plusieurs stabilisants et/ou autres additifs, comme des agents de pénétration, des adhésifs, ou des agents antimottants, colorants, etc..

A titre d'exemple, voici diverses compositions de poudres mouillables (Exemple 23) :

- matière active 25 %
- lignosulfonate de calcium (défloculant) 5 %
- isopropylnaphtalène sulfonate (agent mouillant anionique) 1 %
- silice antimottante 5 %
- kaolin (charge) 64 %

Exemple 24 :

- matière active 80 %
- alkylnaphtalène sulfonate de sodium 2 %
- lignosulfonate de sodium 2 %
- silice antimottante 3 %
- kaolin 13 %

<u>Exemple 25</u> :

| | |
|---|---|
| - matière active | 50 % |
| - alkylnaphtalène sulfonate de sodium | 2 % |
| - méthyl cellulose de faible viscosité | 2 % |
| - terre de diatomées | 46 % |

<u>Exemple 26</u> :

| | |
|---|---|
| - matière active | 90 % |
| - dioctylsulfosuccinate de sodium | 0,2 % |
| - silice synthétique | 9,8 % |

<u>Exemple 27</u> :

| | |
|---|---|
| - matière active | 400 g |
| - lignosulfonate de sodium | 50 g |
| - dibutylnaphtalène sulfonate de sodium | 10 g |
| - silice | 540 g |

<u>Exemple 28</u> :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

<u>Exemple 29</u> :

| | |
|---|---|
| - matière active | 100 g |
| - mélange de sels de sodium de sulfates d'acides gras saturés | 30 g |
| - produit de condensation d'acide naphtalène sulfonique et de formaldéhyde | 50 g |
| - kaolin | 820 g |

Pour obtenir ces poudres à pulvériser ou poudres mouillables, on mélange intimement les matières actives dans des mélangeurs appropriés avec les substances

additionnelles ou on imprègne la matière active fondue sur la charge poreuse et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles de végétaux.

Les granulés "autodispersibles" (en langue anglaise "dry flowable" ; il s'agit plus exactement de granulés facilement dispersibles dans l'eau) ont une composition sensiblement voisine de celle des poudres mouillables. Ils peuvent être préparés par granulation de formulations décrites pour les poudres mouillables, soit par voie humide (mise en contact de la matière active finement divisée avec la charge inerte et avec un peu d'eau, par exemple 1 à 20%, ou de solution aqueuse de dispersant ou de liant, puis séchage et tamisage), soit par voie sèche (compactage puis broyage et tamisage).

A titre d'exemple, voici une formulation de granulé autodispersible (<u>Exemple 30</u>) :

| | |
|---|---|
| - matière active | 200 g |
| - alkylnaphtalène sulfonate de sodium | 20 g |
| - méthylène bis naphtalène sulfonate de sodium | 80 g |
| - kaolin | 700 g |

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type

eau-dans-l'huile ou huile-dans-l'eau et elles peuvent avoir une consistance épaisse comme celle d'une "mayonnaise".

Toutes ces dispersions ou émulsions aqueuses ou bouillies sont applicables aux cultures à désherber par tout moyen convenable, principalement par pulvérisation, à des doses qui sont généralement de l'ordre de 50 à 500 litres de bouillie à l'hectare.

Les granulés destinés à être disposés sur le sol sont habituellement préparés de manière qu'ils aient des dimensions comprises entre 0,1 et 2 mm et ils peuvent être fabriqués par agglomération ou imprégnation. De préférence, les granulés contiennent 1 à 95 % de matière active et 0 à 50 % d'additifs comme des stabilisants, des agents de modification à libération lente, des liants et des solvants.

Selon un exemple de composition de granulé, on utilise les constituants suivants (Exemple 31) :

- matière active 50 g
- propylène glycol 25 g
- huile de lin bouillie 50 g
- argile (granulométrie : 0,3 à 0,8 mm) 910 g

La présente invention concerne aussi un procédé de désherbage qui consiste à appliquer aux plantes devant être détruites une quantité efficace d'un composé de formule (I).

Les produits et compositions selon l'invention s'appliquent commodément sur la végétation et notamment sur les mauvaises herbes à éliminer lorsque celles-ci présentent un feuillage vert. On peut également opérer de manière à ce que la culture soit semée avant ou après traitement.

Pour les composés adaptés à cette application, on peut aussi utiliser un procédé de désherbage qui consiste à appliquer une quantité efficace d'un composé de formule (I) sur des zones ou terrains où l'on veut empêcher la pousse ou le développement de plantes n'ayant pas encore poussé (application de préémergence).

La dose d'application de matière active est généralement comprise entre 1 et 1000 g/ha, de préférence entre 5 et 250 g/ha (généralement 50 à 500 litres de bouillie à l'hectare).

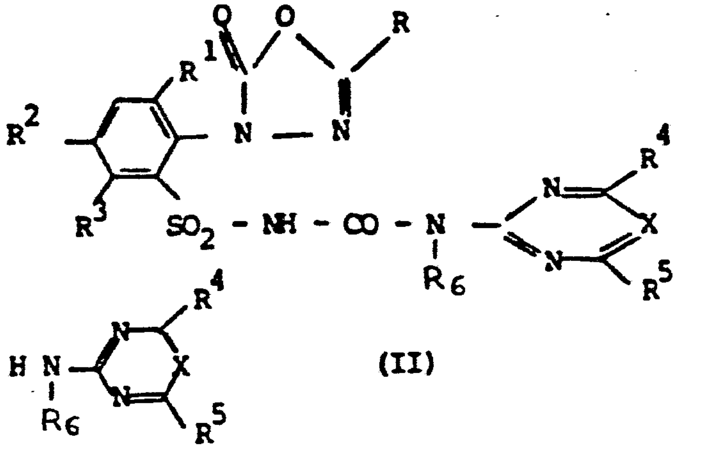

(I)

(II)

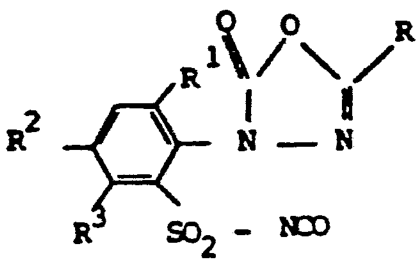

(III)

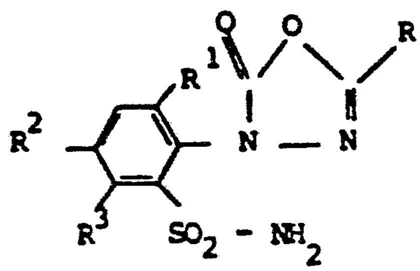

(IV)

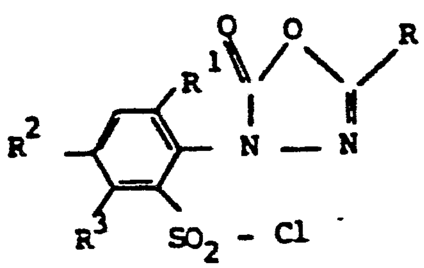

(V)

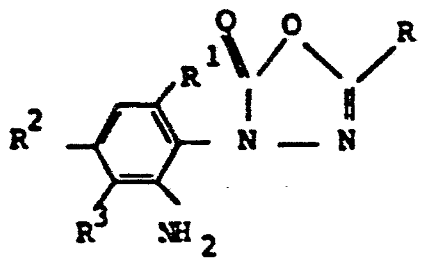

(VI)

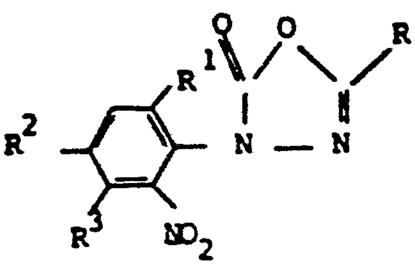

(VII)

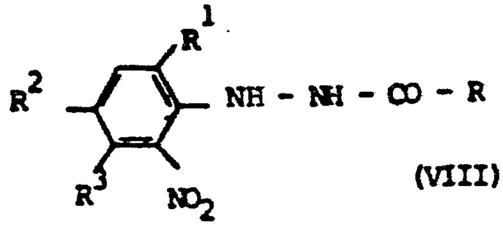

(VIII)

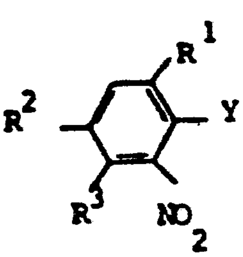

(IX)

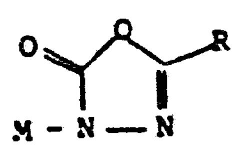

(X)

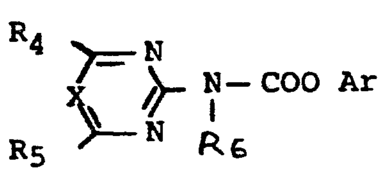

(XI)

## REVENDICATIONS

1) Composés à groupe sulfonylurée utilisables notamment comme herbicides, caractérisés en ce qu'ils ont pour formule la formule (I) présentée dans la description dans laquelle :

R est l'atome d'hydrogène ou un radical alkyle inférieur, alcényle inférieur, alcynyle inférieur, alcoxy inférieur, alcoxy alkyle inférieur, halogénoalkyle inférieur ;

$R_1$, $R_2$, $R_3$ représentent un atome d'hydrogène ou d'halogène ou un radical alkyle inférieur, alcoxy inférieur, halogénoalkyle inférieur, alcoxycarbonyle inférieur ;

X est un radical trivalent : -CH = ou - N =

$R_4$, $R_5$ identiques ou différents représentent un radical alkyle inférieur (de préférence méthyle), alcoxy inférieur (de préférence méthoxy), halogénoalkyle inférieur, halogène (de préférence le chlore)

$R_6$ est H ou alkyle inférieur, de préférence H.

2) Composés selon la revendication 1, caractérisés en ce que $R_1$, $R_3$ correspondent à l'atome d'hydrogène.

3) Composés selon la revendication 1 ou 2, caractérisés en ce que R est l'atome d'hydrogène ou un radical alcoxy alkyle inférieur (de préférence méthoxy méthyle) ou alcoxy inférieur (de préférence méthoxy) et X est -CH =.

4) Composés selon lune des revendications 1 à 3, caractérisés en ce que $R_2$ est un atome d'hydrogène, un atome d'halogène (de préférence le chlore), un radical alcoxy inférieur (de préférence méthoxy) et X est -CH=.

5) Composés selon l'une des revendications 1 à 4, caractérisés en ce que $R_4$, $R_5$, identiques ou différents, sont un atome d'halogène ou un radical alkyle ou alkoxy (de préférence méthyle ou méthoxy).

6) Composés selon la revendication 5, caractérisés en ce que $R_5$ est un radical alkyle ou alcoxy (de préférence méthyle ou méthoxy).

7) Composés selon la revendication 1 ou 2, caractérisés en ce que $R_1$, $R_2$ sont l'atome d'hydrogène et X est -N=.

8) Composés selon lune des revendications 1, 2 ou 7, caractérisés en ce que $R_4$ est un radical alkyle inférieur (de préférence méthyle) et $R_5$ un radical alcoxy inférieur (de préférence méthoxy).

9) Composés caractérisés en ce qu'ils ont l'une des formules (III) à (VII) présentées dans la description et dans lesquelles les divers substituants ont les significations données dans l'une des revendications 1 à 8.

10)Composés caractérisés en ce qu'ils ont l'une des formules (VIII) à (IX) présentées dans la description et dans lesquelles les divers substituants ont les significations données dans l'une des revendications 1 à 8.

11)Procédé de préparation de composés selon l'une des revendications 1 à 8, caractérisé en ce que un sulfonamide de formule (IV) est transformé en sulfonylisocyanate de formule (III) par action de phosgène et que ce dernier est transformé en produit de formule (I) par action d'un composé aminé de formule (II), les diverses formules (I), (II), (III) et (IV) étant celles présentées dans la description.

12)Procédé de préparation des composés selon l'une des revendications 1 à 8, caractérisé en ce que les sulfonamides de formules (IV) sont transformés directement en sulfonylurée de formule (I) par action d'un pyrimidinyl- ou triazinyl carbamate d'aryle (XI), (de préférence phényle), de préférence en quantité stoechiométrique en présence d'une amine tertiaire (par exemple le diaza-1,8 bicyclo [5,4,0] undecène -7) de préférence également en quantité stoechiométrique dans

un solvant inerte tel que chloroforme, acétonitrile, acétone, tétrahydrofuranne ou dioxanne à une température allant de 20 à 100°.

13)Procédé de préparation de composés de formule (IV) telle que présentée dans la description, caractérisé en ce qu'on fait réagir l'ammoniac sur un chlorure de sulfonyle de formule (V) telle que présentée dans la description, les divers substituants dans ces formules (IV) et (V) ayant l'une ou l'autre des significations indiquées dans l'une des revendications 1 à 8.

14)Procédé de préparation de produit de formule (VI) telle que présentée dans la description, caractérisé en ce qu'on opère une réduction ou une hydrogénation d'un composé de formule (VII) telle que présentée dans la description, les divers substituants dans ces formules (VI) et (VII) ayant l'une ou l'autre des significations indiquées dans l'une des revendications 1 à 8.

15)Procédé de préparation de composés de formule (VII) telle que présentée dans la description, caractérisé en ce que l'on fait réagir du phosgène sur un composé de formule (VIII) telle que présentée dans la description, les divers substituants dans ces formules (VII) et (VIII) ayant l'une ou l'autre des significations indiquées dans l'une des revendications 1 à 8.

16)Procédé de préparation de composés de formule (VII) telle que présentée dans la description, caractérisé en ce que l'on fait réagir un composé de formule (IX) telle que présentée dans la description avec un composé de formule (X) telle que présentée dans la description dans lesquelles le symbole Y représente un atome d'halogène, de préférence F ou Cl , le symbole M représente un atome de métal alcalin, de préférence le lithium, le sodium ou le potassium, les autres substituants ayant l'une ou l'autre des significations indiquées dans l'une des revendications 1 à 4, ces diverses formules (VII), (IX) et (X) étant celles présentées dans la description.

17)Utilisation des composés selon l'une des revendications 1 à 8 à titre d'herbicides, notamment sous forme de compositions herbicides, contenant comme matière active un composé selon l'une des revendications 1 à 8, cette matière active étant en association avec au moins un support inerte, usuel, acceptable en agriculture.

18)Compositions selon la revendication 17, caractérisées en ce qu'elles contiennent 0,05 à 95 % de matière active.

19)Compositions selon la revendication 18, caractérisées en ce qu'elles contiennent 5 à 50 % d'agent tensioactif.

20)Procédé pour le désherbage, caractérisé en ce qu'on applique au contact des feuilles des végétaux à supprimer ou sur le sol une dose efficace d'une matière active selon l'une des revendications 1 à 8 ou d'une composition selon l'une des revendications 17 à 19.

21)Procédé selon la revendication 20, caractérisé en ce qu'on applique une composition selon l'une des revendications 17 à 19, la matière active étant appliquée à raison de 1 à 1000 g/ha, de préférence de 5 à 250 g/ha.

## REVENDICATIONS POUR L'AUTRICHE, L'ESPAGNE ET LA GRECE

1)Composition herbicide à usage agricole, caractérisée en ce qu'elle comprend comme matière active un composé à groupe sulfonylurée ayant pour formule la formule (I) présentée dans la description dans laquelle :

R est l'atome d'hydrogène ou un radical alkyle inférieur, alcényle inférieur, alcynyle inférieur, alcoxy inférieur, alcoxy alkyle inférieur, halogénoalkyle inférieur ;

$R_1$, $R_2$, $R_3$ représentent un atome d'hydrogène ou d'halogène ou un radical alkyle inférieur, alcoxy inférieur, halogénoalkyle inférieur, alcoxycarbonyle inférieur ;

X est un radical trivalent : -CH = ou - N =

$R_4$, $R_5$ identiques ou différents représentent un radical alkyle inférieur (de préférence méthyle), alcoxy inférieur (de préférence méthoxy), halogénoalkyle inférieur, halogène (de préférence le chlore)

$R_6$ est H ou alkyle inférieur, de préférence H.

2) Composition selon la revendication 1, caractérisée en ce que $R_1$, $R_3$ correspondent à l'atome d'hydrogène.

3) Composition selon la revendication 1 ou 2, caractérisée en ce que R est l'atome d'hydrogène ou un radical alcoxy alkyle inférieur (de préférence méthoxy méthyle) ou alcoxy inférieur (de préférence méthoxy) et X est -CH =.

4) Composition selon l'une des revendications 1 à 3, caractérisée en ce que $R_2$ est un atome d'hydrogène, un atome d'halogène (de préférence le chlore), un radical alcoxy inférieur (de préférence méthoxy) et X est -CH=.

5) Composition selon l'une des revendications 1 à 4, caractérisée en ce que $R_4$, $R_5$, identiques ou différents, sont un atome d'halogène ou un radical alkyle ou alkoxy (de préférence méthyle ou méthoxy).

6) Composition selon la revendication 5, caractérisée en ce que $R_5$ est un radical alkyle ou alcoxy (de préférence méthyle ou méthoxy).

7) Composition selon la revendication 1 ou 2, caractérisée en ce que $R_1$, $R_2$ sont l'atome d'hydrogène et X est -N=.

8) Composition selon lune des revendications 1, 2 ou 7, caractérisée en ce que $R_4$ est un radical alkyle inférieur (de préférence méthyle) et $R_5$ un radical alcoxy inférieur (de préférence méthoxy).

9) Composition caractérisee en ce qu'elle a l'une des formules (III) à (VII) présentées dans la description et dans lesquelles les divers substituants ont les significations données dans l'une des revendications 1 à 8.

10)Composition caractérisée en ce qu'elle a l'une des formules (VIII) à (IX) présentées dans la description et dans lesquelles les divers substituants ont les significations données dans l'une des revendications 1 à 8.

11)Procédé de préparation de composés de formule (I) selon l'une des revendications 1 à 8, caractérisé en ce que un sulfonamide de formule (IV) est transformé en sulfonylisocyanate de formule (III) par action de phosgène et que ce dernier est transformé en produit de formule (I) par action d'un composé aminé de formule (II), les diverses formules (I), (II), (III) et (IV) étant celles présentées dans la description.

12)Procédé de préparation des composés de formule (I) selon l'une des revendications 1 à 8, caractérisé en ce que les sulfonamides de formules (IV) sont transformés directement en sulfonylurée de formule (I) par action d'un pyrimidinyl- ou triazinyl carbamate d'aryle (XI), (de préférence phényle), de préférence en quantité

stoechiométrique en présence d'une amine tertiaire (par exemple le diaza-1,8 bicyclo [5,4,0] undecène -7) de préférence également en quantité stoechiométrique dans un solvant inerte tel que chloroforme, acétonitrile, acétone, tétrahydrofuranne ou dioxanne à une température allant de 20 à 100°.

13)Procédé de préparation de composés de formule (IV) telle que présentée dans la description, caractérisé en ce qu'on fait réagir l'ammoniac sur un chlorure de sulfonyle de formule (V) telle que présentée dans la description, les divers substituants dans ces formules (IV) et (V) ayant l'une ou l'autre des significations indiquées dans l'une des revendications 1 à 8.

14)Procédé de préparation de produit de formule (VI) telle que présentée dans la description, caractérisé en ce qu'on opère une réduction ou une hydrogénation d'un composé de formule (VII) telle que présentée dans la description, les divers substituants dans ces formules (VI) et (VII) ayant l'une ou l'autre des significations indiquées dans l'une des revendications 1 à 8.

15)Procédé de préparation de composés de formule (VII) telle que présentée dans la description, caractérisé en ce que l'on fait réagir du phosgène sur un composé de formule (VIII) telle que présentée dans la description, les divers substituants dans ces formules (VII) et (VIII) ayant l'une ou l'autre des significations indiquées dans l'une des revendications 1 à 8.

16)Procédé de préparation de composés de formule (VII) telle que présentée dans la description, caractérisé en ce que l'on fait réagir un composé de formule (IX) telle que présentée dans la description avec un composé de formule (X) telle que présentée dans la description dans lesquelles le symbole Y représente un atome d'halogène, de préférence F ou Cl , le symbole M représente un atome de métal alcalin, de préférence le lithium, le sodium ou

le potassium, les autres substituants ayant l'une ou l'autre des significations indiquées dans l'une des revendications 1 à 4, ces diverses formules (VII), (IX) et (X) étant celles présentées dans la description.

17)Procédé de traitement des végétaux contre les mauvaises herbes caractérisé en ce que il consiste à appliquer sur ces végétaux une dose efficace d'une composition selon l'une des revendications 1 à 10.